# EUROPEAN PATENT SPECIFICATION

(11) **EP 3 932 339 B1**
(45) Date of publication and mention of the grant of the patent: **17.01.2024**
(21) Application number: 21181925.5
(22) Date of filing: 28.06.2021
(51) Int. Cl.: A61B 17/22, A61F 2/958, A61M 25/10, A61B 17/00, A61B 17/12

(54) **ISOLATED STENTING WITH DUAL LUMEN ASPIRATION**
ISOLIERTES STENTING MIT DOPPELLUMENABSAUGEN
ENDOPROTHÈSE ISOLÉE AVEC ASPIRATION À DOUBLE LUMIÈRE

(30) Priority: 29.06.2020 US 202016914881
(43) Date of publication of application: 05.01.2022
(73) Proprietor: Neuravi Limited, Galway H91 K5YD (IE)
(72) Inventor: KEATING, Karl, Galway, H91 K5YD (IE); KELLY, Ronald, Galway, H91 K5YD (IE)
(74) Representative: Carpmaels & Ransford LLP

(56) References cited:
- EP-A1- 3 473 195
- US-A1- 2005 256 565
- US-A1- 2006 200 191
- US-A1- 2017 281 140
- US-B1- 6 485 500

## Description

### FIELD OF INVENTION

The present invention generally relates to intravascular treatment systems and methods for constructing an intravascular treatment systems, and more particularly, to systems for treating intravascular lesions such as lesions relating to intracranial atherosclerosis disease (ICAD).

### BACKGROUND

Atherosclerosis results from lesions which narrow and reduce the space in the lumen of vessels in the vasculature. Such lesions are usually composed of plaque, which can be fat, cholesterol, calcium, or other components of the blood. Severe occlusion or closure can impede the flow of oxygenated blood to different organs and parts of the body and result in other cardiovascular disorders such as heart attack or stroke. Narrowing of vessels, or stenosis, increases the risk that clots and other emboli can lodge at such locations, especially in the neurovascular where vessel diameters are already small. ICAD is the narrowing of those arteries and vessels supplying blood to the brain and represents the most common proximate mechanism of ischemic stroke.

Treatment for vascular occlusions can include utilizing drugs, such as anticoagulants or anti-platelet agents, as well as medical procedures such as surgical endarterectomy, angioplasty, and stenting. Much of the recent success in endovascular revascularization treatments (ERT) has been the further development of safe thrombectomy devices. Devices such as stentrievers, direct-aspiration systems, and other clot retrieval devices have been strongly associated with better clinical outcomes. However, these devices are primarily designed to recanalize the vessel by removing and retrieving an occluding embolus. Sufficient recanalization may not occur if there is also significant stenosis present at the occlusion site, increasing the need for implanted stents.

Treatment methods for addressing clots and lesions in the neurovascular in particular depend on the degree of stenosis, the shape of the target occlusion site (i.e. truncal, branching, etc.), and the patient's overall condition. For example, mechanical procedures often involve using medical devices to retrieve an occlusive clot and then utilizing balloons and stents to open a narrowed artery. Following the use of a stentriever or other clot retrieval device, a balloon is delivered to a target site and inflated to dilate the stenosis. The balloon can then be removed and exchanged through a catheter for a stent delivery device. If desirable, once the stent is in place a balloon can be inflated inside the stent to press the struts of the stent scaffold frame firmly against the inner wall of the vessel.

In the case of ICAD, prolonging treatment and/or crossing the occlusion with multiple devices can increase the likelihood that the ICAD ruptures or fragments. Such fragments can include but are not limited to blood clots, plaque, and other thrombi debris. The fragments can lead to vascular occlusions causing extensive stroke or death. Due to the difficulty in diagnosing ICAD, a physician may need to cross the lesion multiple times during a treatment, thereby further increasing the likelihood of rupture or fragmenting.

There therefore remains a need for systems and devices to continue to address and improve treatments for intravascular occlusions, specifically ICAD.

US 6485500 B1 relates to an emboli protection system that provides one or more inflatable blocking balloons for isolation of a section of a blood vessel to prevent migration of emboli from the section during an interventional procedure, and fluid infusion and evacuation ports for flushing emboli from the isolated section. There is provided a distal blocking balloon catheter, over which an interventional device can be introduced, and a proximal blocking balloon catheter to be introduced over the interventional device for isolating a portion of a blood vessel to be treated.

US 2006/200191 A1 relates to a guidewire having an occlusive device such as balloon or a filter at one end that is advanced across the occlusion using a guide catheter, and the occlusive device is expanded distal to the occlusion to occlude the blood vessel.

US 2005/256565 A1 relates to a catheter having a distal end portion for being guided through a vessel to the site of an irregularity. A balloon associated with said distal end portion of the catheter for selective inflating to contact the walls of the vessel urges a stent carried by the distal end portion of the catheter in a constricted condition for passage through the vessel into an expanded form with the stent spanning the afflicted wall portion and contacting the adjacent wall portions.

### SUMMARY

The invention is defined in the independent claims. Embodiments of the invention are defined in the dependent claims. It is an object of the present invention to provide a catheter-based system for isolated stenting of an intravascular lesion that can include two expandable occlusion devices with a balloon expandable stent or self-expanding stent therebetween.

Certain surgical methods are described with reference to the system according to the present invention. No claim is directed to these methods but the system is capable of being used and is intended to be used in such methods.

Expandable occlusion devices can be expanded in a distal direction and a proximal direction in relation to the lesion to occlude vasculature. A treatment agent can be injected into a cavity formed between the expandable occlusion devices. A coating can be applied to one or both occlusion devices to prevent adherence to the treatment agent. The stent can be deployed across the lesion while the occlusion devices are in place. Fragments dislodged during stenting can be aspirated.

An example intravascular treatment system includes a balloon guide catheter and a delivery tube. The balloon guide catheter includes a distal and proximal end. A balloon is positioned approximate to the distal end. The balloon guide catheter includes multiple lumens, including an inflation lumen in communication with the balloon, drug delivery lumen, and a device delivery lumen. The delivery tube is sized to be positioned within the device delivery lumen. An expandable occlusion element is disposed on the delivery tube.

The system also includes a stent surrounding the delivery tube. The stent is positioned in a proximal direction in relation to the expandable occlusion element.

The system also includes a sheath surrounding the stent. In one example, the stent can be self-expanding. The sheath is positioned to inhibit the stent from expanding and is moveable to allow the stent to expand.

The expandable occlusion element can be self-expandable. The sheath is positioned to inhibit the expandable occlusion element from expanding and is moveable to allow the expandable occlusion element to expand. Alternatively, the expandable occlusion element can be inflatable.

The delivery tube can further include an angioplasty balloon. The stent can surround the angioplasty balloon.

In one example, the delivery tube and the balloon disposed on the balloon guide catheter can each have a coating to effectively inhibit adherence with an intravascular lesion sealing agent.

In one example, the drug delivery lumen can have a coating therein to inhibit adherence with an intravascular lesion sealing agent. The coating can include low friction materials, such as PTFE, plasma treatment, fluoropolymers, and other low friction polymers.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1A is an illustration of an intravascular treatment system during a treatment step, according to aspects of the present invention;
FIGs. 1B and 1C are cross-sectional views of the treatment system as indicated in FIG. 1A;
FIGs. 2A through 2J are a sequence of illustrations depicting steps of treatment of an intravascular lesion using the treatment system illustrated in FIGs. 1A through 1C, according to aspects of the present invention;
FIG. 3A and 3D are a sequence of illustrations depicting steps of treatment of an intravascular lesion using an alternative treatment system, according to aspects of the present invention;
FIGs. 3B and 3C are cross-sectional views of the treatment system as indicated in FIG. 3A;
FIGs. 4A through 4C are a sequence of illustrations depicting steps of treatment of an intravascular lesion using another alternative treatment system, according to aspects of the present invention;
FIGs. 5A through 5C illustrate variations of a cross-section of a catheter of an intravascular treatment system, according to aspects of the present invention;
FIG. 6 is a flow diagram outlining steps for treating an intravascular lesion, according to aspects of the present invention; and
FIG. 7 is a flow diagram outlining steps for constructing an intravascular treatment system, according to aspects of the present invention.

### DETAILED DESCRIPTION

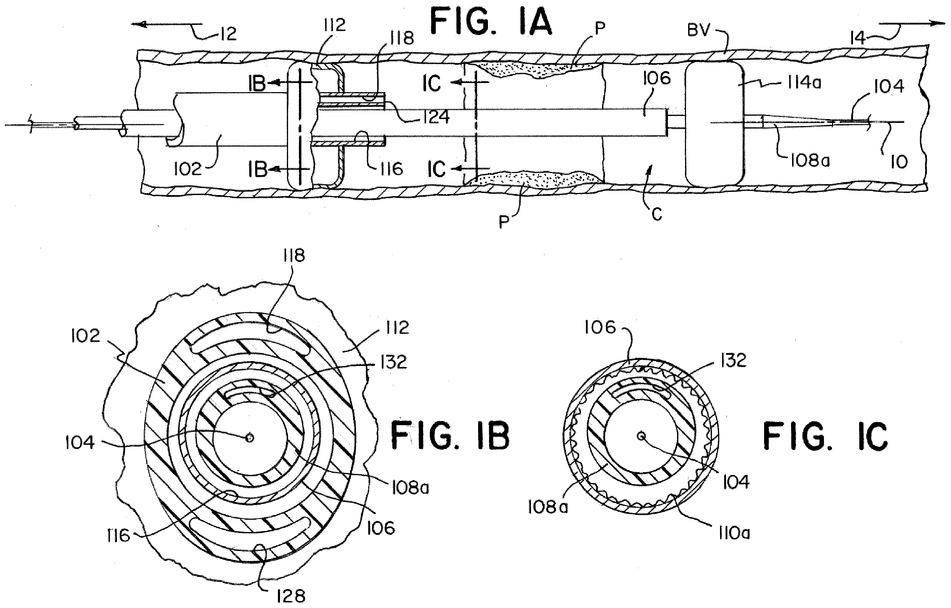

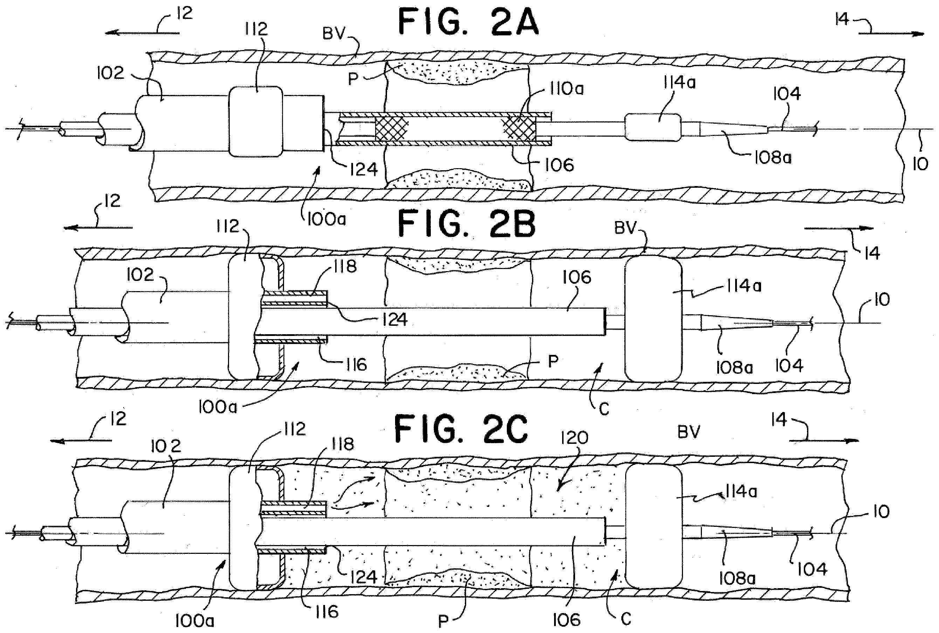

FIG. 1A is an illustration of an intravascular treatment system 100a during treatment of a lesion P in a blood vessel BV. FIGs. 1B and 1C are cross-sectional views of the treatment system 100a as indicated in FIG. 1A. FIGs. 2A through 2J illustrate treatment steps using the treatment system 100a illustrated in FIG. 1. Referring collectively to FIGs. 1A through 1C and FIGs. 2A through 2J, the treatment system 100a can include a balloon guide catheter 102 and a delivery tube 108a with a stent 110a carried over the delivery tube 108a. The system 100a can be configured to create an isolated cavity C within a blood vessel BV and deliver the stent 110a and/or other treatments such as drugs and aspiration to a lesion P within the isolated cavity C. The system 100a is elongated along a longitudinal axis 10.

The balloon guide catheter 102 can include a distal end 124. The balloon guide catheter 102 can further include an expandable element 112 (hereinafter "proximal balloon") positioned approximate to the distal end 124 of the balloon guide catheter 102.

The proximal balloon 112 can have a collapsed configuration and an expanded configuration. In the collapsed configuration, the expandable material of the proximal balloon 112 can be collapsed around the balloon guide catheter 102 to allow the balloon guide catheter 102 to traverse vasculature. In the expanded configuration, the proximal balloon 112 can be inflated via the inflation lumen 128. The proximal balloon 112 can be expanded such that the proximal balloon 112 apposes the inner walls of the blood vessel BV.

Alternatively, the proximal expandable element 112 can be made of porous material. The pores of the proximal expandable element 112 can be sized to inhibit dislodged fragments F from the lesion P from passing through the proximal expandable element 112. The pores of the proximal expandable element 112 can be sized to allow the passage of blood.

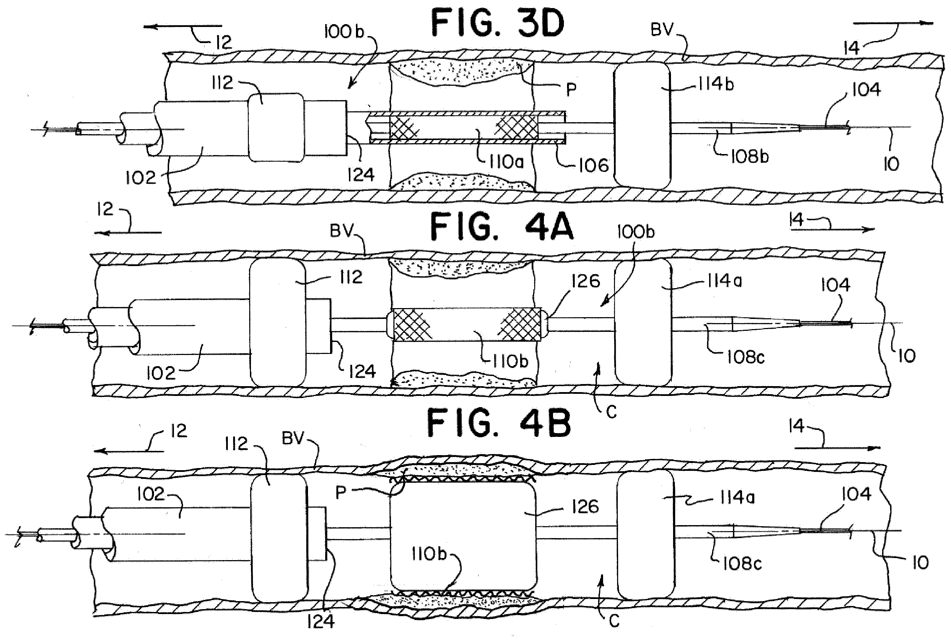

The balloon guide catheter 102 can include one or more lumens. Each lumen can be used independently of one another at the same or different time. Each lumen 116, 118, 128 can facilitate multiple tasks, including aspiration and delivery of drugs and/or components of the systems 100a-c illustrated and described herein. In one example, each lumen can be used simultaneously to perform different treatment steps. The balloon guide catheter 102 can include a drug delivery lumen 118. The drug delivery lumen 118 can enable users of the system 100a to deliver drugs, including ICAD treatment agents 120, through the system 100a directly to the desired location. The drug delivery lumen 118 can include low friction materials and/or standard materials with one or more liners. The liners can include PTFE, polyimide, HDPE, and other similar low friction polymers. Alternatively, or in addition to, the drug delivery lumen 118 can have one or more coatings of material to inhibit the ICAD treatment agent 120 from adhering to the drug delivery lumen 118. The coating of the drug delivery lumen 118 can facilitate delivery of the ICAD treatment agent 120 and prevent residual amounts of the ICAD treatment agent 120 from remaining within the drug delivery lumen 118. The coatings can include low friction materials, including PTFE, plasma treatment, fluoropolymers, and other low friction polymers. The balloon guide catheter 102 can also include a device delivery lumen 116. The device delivery lumen 116 can facilitate delivery of components sized to fit within the device delivery lumen 116, such as the delivery tube 108, to the appropriate location within the vasculature. In one example, the device delivery lumen 116 can be used to aspirate. The balloon guide catheter 102 can also include an inflation lumen 128, as illustrated in FIG. 4B. The inflation lumen 128 can be in fluid communication with the proximal balloon 112 and can used to inflate and deflate the proximal balloon 112. The balloon guide catheter 102 can be sufficiently long such that a proximal end of the balloon guide catheter 102 can be positioned outside the patient when the treatment system 100a is positioned at the lesion P. Configured as such, a user (e.g. physician) can manipulate the proximal end of the balloon guide catheter 102 to position the distal end 124 of the balloon guide catheter 102.

The delivery tube 108a can be positioned within the device delivery lumen 116 of the balloon guide catheter 102. The delivery tube 108a can be sized to be translatable through the device delivery lumen 116 of the balloon guide catheter 102. The delivery tube 108a can be advanced through the balloon guide catheter 102 and extended in a distal direction 14 from the distal end 124 of the balloon guide catheter 102. The delivery tube 108a can further extend in a distal direction 14 from the lesion P when placed across the lesion P.

The delivery tube 108a can include a distal balloon 114a. The distal balloon 114a can be expandable such that the distal balloon 114a can appose the inner walls of the blood vessel BV. The delivery tube 108a can include an inflation lumen 132 to inflate the distal balloon 114a.

The system 100a can include a stent 110a surrounding the delivery tube 108a, as illustrated in cross section in FIG. 1C and in profile in FIG. 2A. The stent 110a can be positioned in a proximal direction 12 from the distal balloon 114a on the delivery tube 108a. When the system 100 is placed for treatment, the stent 110a can be positioned in a distal direction 14 from the proximal balloon 112. The system 100a can include a sheath 106 surrounding the delivery tube 108a and the stent 110a. The sheath can inhibit the stent 110a from self-expanding.

The proximal balloon 112 can be positioned in a proximal direction 12 from the distal balloon 114a. An isolated cavity C can be created between the proximal balloon 112 and the distal balloon 114a. The isolated cavity C can contain the lesion P. When the proximal balloon 112 and the distal balloon 114a are in an expanded configuration, the proximal balloon 112 and the distal balloon 114a can prevent fragments F from the lesion P from migrating away from the lesion P.

The system 100a can be delivered over a guidewire 104. The guidewire 104 can facilitate manipulating the system 100a to the site of the lesion P.

FIGs. 2A through 2J are a sequence of illustrations depicting steps of treatment of an intravascular lesion P using the treatment system 100a including a distal balloon 114a on the delivery tube 108a.

In FIG. 2A, the intravascular treatment system 100a can be advanced through the vasculature to the lesion P. To reach the position illustrated in FIG. 2A, first a guidewire 104 can be extended through vasculature of the patient and positioned across the lesion P, then the balloon guide catheter 102 and the delivery tube 108a can be translated in the distal direction 14 over the guidewire 104 to the illustrated position. The balloon guide catheter 102 can be advanced until the proximal balloon 112 is in a proximal direction 12 from the lesion P. The delivery tube 108a including the distal balloon 114a in a collapsed configuration can then be translated through the device delivery lumen 116 of the balloon guide catheter 102 and extended from the distal end 124 of the catheter 102. The delivery tube 108a can be extended until the collapsed distal balloon 114a is in a distal direction 14 from the lesion P. The stent 110a in a collapsed configuration can be positioned between the distal balloon 114a and the proximal balloon 112. A sheath 106 can be positioned over the stent 110a while the intravascular treatment system 100a is being positioned within the blood vessel BV. The sheath 106 can prevent the stent 110a from self-expanding at an undesired time or location.

In FIG. 2B, the distal balloon 114a can be inflated to circumferentially appose walls of the blood vessel BV. Similarly, the proximal balloon 112 can be inflated via the inflation lumen 128 of the balloon guide catheter 102 to circumferentially appose the walls of the blood vessel BV. The expanded distal balloon 114a and the expanded proximal balloon 112 can create an isolated cavity C within the blood vessel BV. The distal balloon 114a can be configured to inflate before, after, or simultaneously with the proximal balloon 112.

In FIG. 2C, an ICAD treatment agent 120 can be released into the isolated cavity C. The ICAD treatment agent 120 can be delivered through the drug delivery lumen 118 of the balloon guide catheter 102. The balloons 112, 114a can inhibit the treatment agent 120 from migrating away from the treatment area by confining the treatment agent to the cavity C.

The ICAD treatment agent 120 can be one or more drugs or fluids designed to stabilize the lesion P and/or form a seal against the lesion P. In one example, the ICAD treatment agent 120 can be one or more antiplatelet and/or anticoagulant drugs. The ICAD treatment agent 120 can be a flexible sealing material that can alter shape upon treatment of the lesion P through angioplasty and/or stenting. Further, the ICAD treatment agent 120 can facilitate maintaining open capillary pathways. In one example, the ICAD treatment agent 120 can prevent thrombosis locally by being delivered to a particular location within the blood vessel BV and confined to such particular location through expanded occlusion elements 112, 114a, as illustrated in FIG. 2C. The ICAD treatment agent 120 can be released over a predetermined time period. The time period over which an ICAD treatment agent 120 can be released can be based on the size of the lesion P, location of the lesion P, type of ICAD treatment agent, and other factors. In one example, the ICAD treatment agent 120 can be released when the system 100a comes into contact with a ruptured lesion P. Once a lesion P ruptures, the ICAD treatment agent 120 can be released and allowed to form a seal on the exterior surface of the remaining part lesion P in the blood vessel BV. In another example, the ICAD treatment agent 120 can be released when blood flow becomes stagnant or minimal.

In some treatments, aspiration can be applied through the aspiration lumen 116 of the balloon guide catheter 102 to prevent pressure buildup in the cavity C due to the addition of the treatment agent 120.

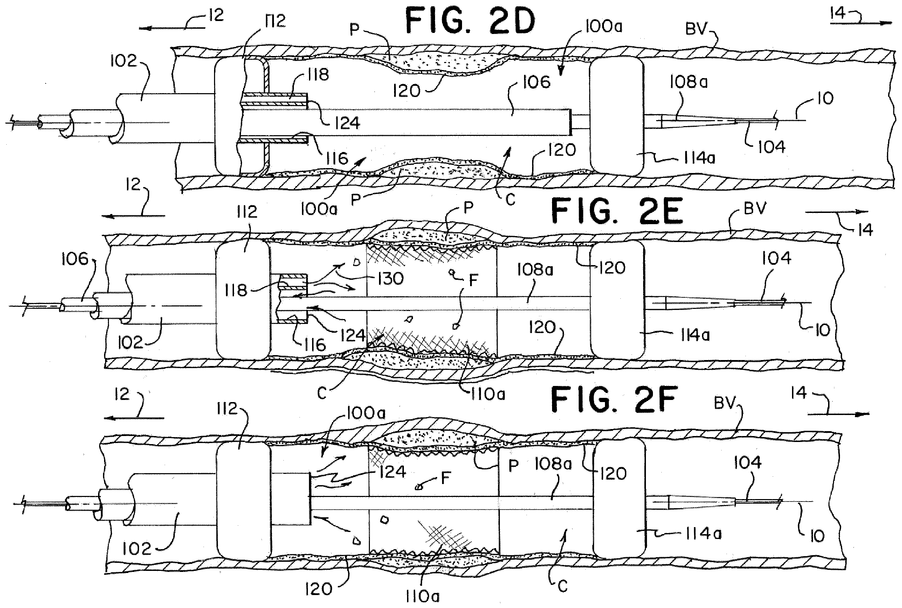

In FIG. 2D, the ICAD treatment agent 120 can adhere to the exterior surface of the lesion P, forming a seal. The ICAD treatment agent 120 can additionally adhere to the inner walls of the blood vessel BV in one example. Alternatively, the ICAD treatment agent 120 can be designed such that the agent 120 does not adhere to the endothelial layers of the blood vessel BV. The seal of ICAD treatment agent 120 on the lesion P can be flexible, such that the seal can alter shapes during intravascular treatment steps, including inflating and deflating an angioplasty balloon 126 and/or expanding a stent 110a, 110b across the lesion P. The isolated cavity C can be aspirated via an aspiration lumen 116. In one example, the device delivery lumen 116 can serve as the aspiration lumen when an aspiration source is connected to the system 100a.

In some treatments, saline solution and/or ICAD treatment agent 120 can be supplied through the drug delivery lumen 118 while aspirating to prevent negative pressure and blood vessel collapse in the cavity C.

In FIG. 2E, the sheath 106 surrounding the stent 110a can be moved in a proximal direction 12 to expose the stent 1 10a. The stent 110a can self-expand within the lumen of the blood vessel BV and press into the plaque of the lesion P. The stent 110a can be made of material capable of self-expanding to an expanded configuration once the sheath 106 is moved in a proximal direction 12. In one example, the stent 110a can be made of shape memory material. Alternatively, or in addition to, the stent 110a can be made of super elastic material. In one example, the superlattice alloy can be Nitinol or an alloy of similar properties. When the stent 110a is in the expanded configuration, the stent 110a can keep the blood vessel BV open.

When the stent 110a presses against the lesion P, fragments F can become dislodged. The expanded distal balloon 114a and expanded proximal balloon 112 can confine the fragments F to within the isolated cavity C and prevent the fragments F from travelling proximally or distally through the blood vessel. When an aspiration is applied, the fragments F can be removed from the blood vessel BV. The device delivery lumen 116 of the balloon guide catheter 102 can be dimensioned to provide passage for fragments F to enter the lumen of the balloon guide catheter 102 while the delivery tube 108a is extended therethrough. In one example, a saline solution 130 can be injected into the isolated cavity C while the stent 110a is expanded across the lumen of the blood vessel BV and across the lesion P. Simultaneously or subsequently, the isolated cavity C can be aspirated through the aspiration lumen 116. In one example, the saline solution can be injected into the isolated cavity C and aspirated at the same rate, thereby maintaining the proximal balloon 112 and the distal balloon 114a in an expanded configuration. By injecting and aspirating the saline solution, fragments of the lesion can continue to become dislodged, and subsequently removed from the blood vessel BV.

In some treatments, saline solution supply rate and aspiration rate can be controlled to manage pressure within the cavity C.

In FIG. 2F, aspiration can continue via the aspiration lumen 116, causing more fragments F of the lesion P to be dislodged. The fragments F dislodged from the lesion P can continue to be confined to the isolated cavity C and removed through the device delivery lumen 116 of the balloon guide catheter 102.

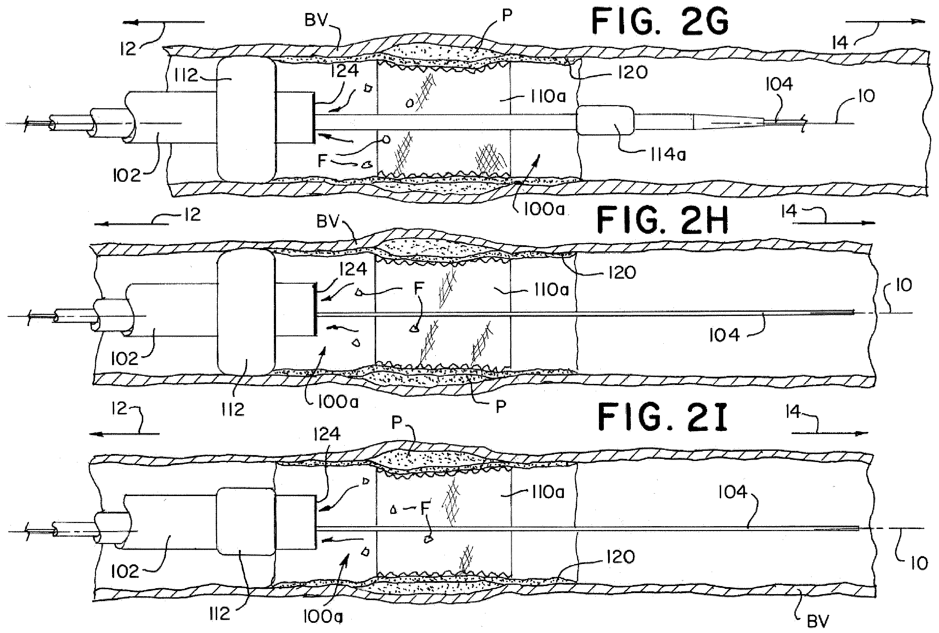

In FIG. 2G, the distal balloon 114a can be deflated, while the proximal balloon 112 can remain inflated. Aspiration of fragments F can continue.

In FIG. 2H, the delivery tube 108a including the deflated distal balloon 114a can be retracted into the device delivery lumen 116 of the balloon guide catheter 102. The expanded stent 110a can remain across the lesion P within the isolated cavity C. Aspiration can continue via the aspiration lumen 116 such that dislodged fragments F of the lesion P can continue to be removed through the lumen of the balloon guide catheter 102. Fragments too large to be aspirated while the delivery tube 108a extends from the distal end 124 of the balloon guide catheter 102 can be aspirated into the balloon guide catheter 102 once the delivery tube 108a is moved proximally into the balloon guide catheter 102.

In FIG. 2I, the proximal balloon 112 can be deflated. The expanded stent 110a can remain across the lesion P. The ICAD treatment agent 120 can remain as a seal along the exterior surface of the remaining lesion P and along the inner walls of the blood vessel BV, allowing the blood vessel BV to remain open.

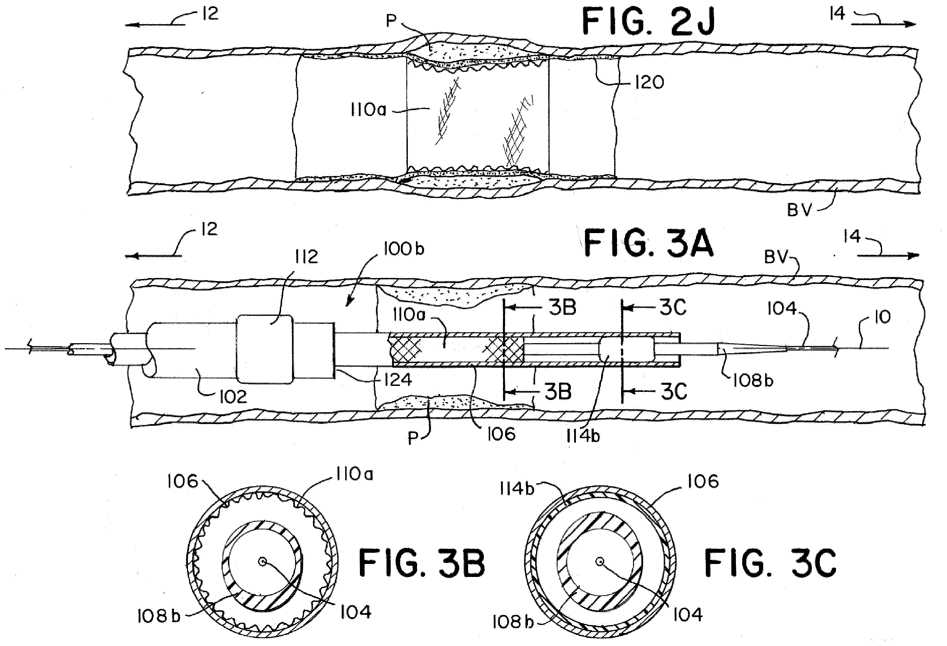

In FIG. 2J, the balloon guide catheter 102 including the proximal balloon 112 can be removed from the blood vessel BV. The expanded stent 110a can be implanted within the blood vessel BV and ICAD treatment agent 120 can remain within the blood vessel BV as a seal around the lesion P and/or inner walls of the blood vessel BV approximate to the lesion P. The combination of the expanded stent 110a and the ICAD treatment 120 seal can keep the blood vessel BV open.

In one example, the stent 110a can be deployed prior to the injection of the ICAD treatment agent 120. Upon expanding the distal balloon 114a and the proximal balloon 112 as described herein, the sheath 106 surrounding the stent 110a can be moved in a proximal direction 12 to expose the stent 1 10a. The stent 110a can self-expand within the lumen of the blood vessel BV and press into the plaque of the lesion P. As the stent 110a presses against the plague of the lesion P, fragments F can become dislodged. The isolated cavity C can be aspirated through the aspiration lumen 116 to remove dislodged fragments F. After aspirating the dislodged fragments F, the ICAD treatment agent 120 can be released into the isolated cavity as described in FIG. 2C. Aspiration via the aspiration lumen 116 can aspirate the ICAD treatment agent 120 and any fragments F dislodged as a result of the release of the ICAD treatment agent 120. Upon aspirating the ICAD treatment agent 120, a saline solution can be released into the isolated cavity C. The distal balloon 114a and the proximal balloon 112 can be deflated, and the stent 110a can remain expanded across the lesion P.

FIGs. 3A and 3D are a sequence of illustrations depicting steps of treatment of an intravascular lesion P using a treatment system 100b having a balloon guide catheter 102 as illustrated in FIGs. 1A through 1C and 2A through 2J and a delivery tube 108b having a similar construction to the delivery tube 108a illustrated in FIGs. 1A through 1C and 2A through 2J with an exception that a distal self-expanding element 114b is used in place of the distal balloon 114a. FIGs. 3B and 3C are cross-sectional illustrations of the system 100b as indicated in FIG. 3A.

FIGs. 3A through 3D illustrate an intravascular treatment system 100b including a self-expanding distal occlusion element 114b disposed on a delivery tube 108b. As the system 100b is placed across the lesion P as illustrated in FIG. 3A, the self-expanding element 114b can be inhibited from expanding by the sheath 106. The stent 110a can also be inhibited from self-expanding by the sheath 106. As illustrated in FIG. 3D, the sheath can be retracted proximally to allow the self-expanding element 114b to circumferentially appose the wall of the blood vessel BV. The self-expanding element can be impermeable to fluids to effectively seal the blood vessel BV when expanded. The distal occlusion element 114b can be made of shape memory material, including shape memory polymers such as polyurethane. Treatment can proceed following the steps illustrated in FIGs. 2B through 2J. The proximal balloon 112 can be inflated before, after, or simultaneously with the expansion of the distal occlusion element 114b. The stent 110a can be self-expandable, and the sheath 106 can be pulled proximally to allow the stent 110a to self-expand. The delivery tube 108b need not include an inflation lumen 132 (see FIG. 1B) when both the stent 110a and distal occlusion element 114b are self-expandable.

FIGs. 4A through 4C are a sequence of illustrations depicting steps of treatment of an intravascular lesion P using another alternative treatment system 100c. The intravascular treatment system 100c illustrated in FIGs. 4A through 4C can have a balloon guide catheter 102 as illustrated in FIGs. 1, 2A through 2J, and 3A through 3B and a delivery tube 108c having a similar construction to the delivery tube 108a illustrated in FIGs. 1 and 2A through 2J with an exception that a balloon expanded stent 110b is used in place of the self-expandable stent 110a. The system 100c need not include a sheath 106 when neither the stent 110b nor the distal balloon 114a rely on the sheath 106 to inhibit respective self-expansion thereof. Alternatively, the intravascular treatment system 100c illustrated in FIGs. 4A through 4C can have a delivery tube 108c having a similar construction to the delivery tube 108b illustrated in FIGs. 3A and 3B with an exception that a balloon expanded stent 110b is used in place of the self-expandable stent 110a. When the system includes a self-expandable distal occlusion element 114b, the system 100c can include a sheath 106 that be positioned to inhibit expansion of the distal occlusion element 114b as illustrated in FIG. 3A that can be moved to allow the distal occlusion element 114b to expand.

The system 100c can further include an angioplasty balloon 126 disposed on the delivery tube 108c. The angioplasty balloon 126 can be disposed on the delivery tube 108c in the proximal direction from the distal balloon 114a.

In FIG. 4A, the system 100c can form an isolated cavity C by expanding the proximal balloon 112 and the distal balloon or self-expanding element 114a, 114b. Expanding the distal occlusion element 114a, 114b can be achieved with steps illustrated in FIGs. 2A and 2B when the distal occlusion element is inflatable and can be achieved with steps illustrated in FIGs. 3A and 3B when the distal occlusion element is self-expandable. The stent 110b need not be sheathed to inhibit expansion. Following the step illustrated in FIG. 4A, the treatment can include delivery and aspiration of a treatment agent 120 similar to as illustrated in FIGs. 2C and 2D,

In FIG. 4B, the angioplasty balloon 126 can be inflated. When the angioplasty balloon is inflated, the stent 110b covering the angioplasty balloon 126 can also be expanded. The angioplasty balloon 126 can be inflated to press the stent 110b into the lesion P. In some treatments, the angioplasty balloon 126 can expand the blood vessel BV. In one example, the angioplasty balloon 126 can include a compliant membrane that allows the angioplasty balloon 126 to expand to a desired size. The angioplasty balloon 126 can further include a non-compliant wrap configured to restrict the expanded size of the angioplasty balloon 126. The angioplasty balloon 126 can be inflated while the proximal balloon 112 and the distal occlusion element 114a, 114b remain inflated.

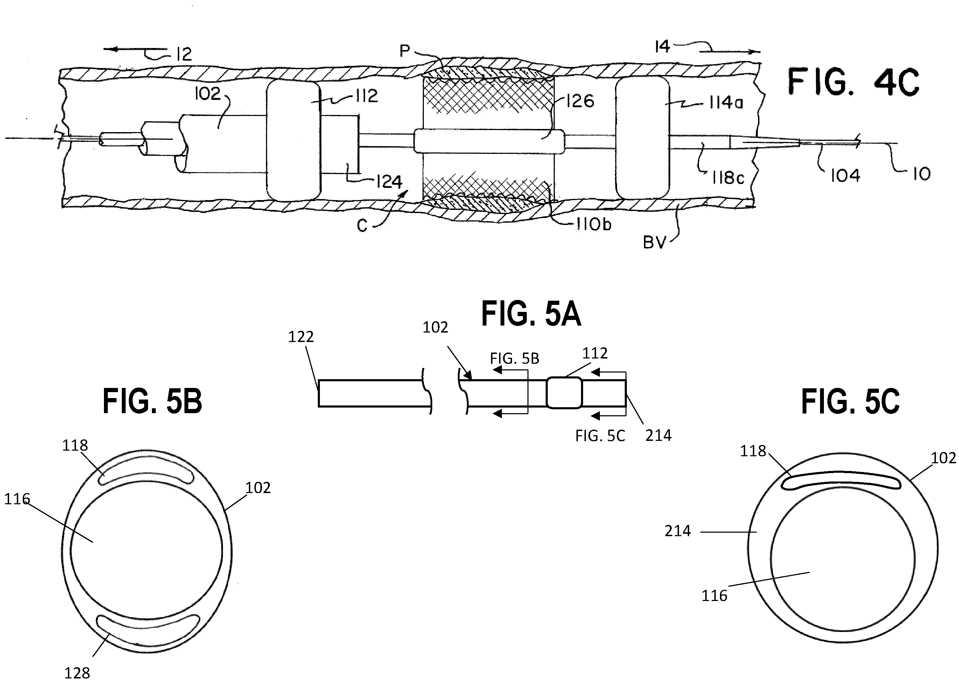

In FIG. 4C, the angioplasty balloon 126 can be deflated while the stent 110b remains expanded against the lesion P and implanted within the blood vessel BV. Inflation and deflation of the angioplasty balloon 126 can dislodge fragments F of the lesion P. The fragments F can be inhibited from flowing from the lesion site P due to the expanded proximal balloon 112 and the distal balloon 114a. Following the step illustrated in FIG. 4C, the treatment steps can proceed as illustrated in FIGs. 2E through 2J.

In one example, the sequence for treating an intravascular lesion P can be conducted in a particular order. In some treatments, the distal occlusion element 114a, 114b and the proximal balloon 112 can be expanded simultaneously. Alternatively, the distal occlusion element 114a, 114b can be first expanded to first block the blood flow downstream, then the proximal balloon 112 can then expanded to block blood flow to the lesion P. Alternatively, the proximal balloon 112 can be inflated first prior to the system 100a crossing the lesion P.

Regardless of the order of expansion of the proximal balloon 112 and distal occlusion element 114a, 114b, once expanded, the proximal balloon 112 and distal occlusion element 114a, 114b can be effective to create the isolated cavity C containing the lesion P. Next, the ICAD treatment agent 120 can be injected into the isolated cavity C via the drug delivery lumen 118. Lastly, the stent 110a, 110b disposed on the delivery tube 108a-c can be expanded into the lesion within the isolated cavity and implanted within the blood vessel BV. The stent can 110a, 110b can press against the plague of the lesion P causing fragment F to become dislodged.

In some treatments, the isolated cavity C can be aspirated before expanding the stent 110a, 110b into the lesion P and after expanding the stent 110a, 110b across the lesion. Alternatively, the isolated cavity C can be aspirated only after expanding the stent 110a, 110b across the lesion P. Alternatively, the isolated cavity C can be aspirated simultaneously while the stent 110a is self-expanding within the blood vessel BV.

In one example, the delivery tube 108a-c and the balloon guide catheter 102 of the intravascular treatment system 100a-c can have one or more coatings of material to inhibit adherence with the ICAD treatment agent 120. Further, in one example, the proximal balloon 112 and the distal occlusion element 114a, 114b can have one or more coatings of material to inhibit adherence with the ICAD treatment agent 120.

FIGs. 5A through 5C illustrates variations of a cross-section of the balloon guide catheter 102 of the intravascular treatment system 100a-c. FIG 4A illustrates a balloon guide catheter 102 having a distal end 124 and a proximal end 122 and a balloon 112 disposed proximate to the distal end 124 of the catheter 102. The balloon guide catheter 102 can include one or more lumens, such that each lumen can facilitate multiple tasks, including aspiration and delivery of drugs and/or components of the system 100a-c.

FIG. 5B illustrates a cross-sectional view of a proximal portion relative to the proximal balloon 112 of the balloon guide catheter 102. The balloon guide catheter 102 can include a device delivery lumen 116, a drug delivery lumen 118, and an inflation lumen 128. The delivery tube 108a-c can be sized to fit within the device delivery lumen 116. The device delivery lumen 116 can also be the aspiration lumen. The drug delivery lumen 118 can facilitate delivery of the ICAD treatment agent 120 and saline solution to the isolated cavity C within the blood vessel BV. The inflation lumen 128 be in fluid communication with the proximal balloon 112 and be configured to inflate and deflate the proximal balloon 112.

FIG. 5C illustrates a cross-sectional view of a distal end 124 of the balloon guide catheter 102. At the distal end 124, the balloon guide catheter 102 can include a device delivery lumen 116 and a drug delivery lumen 118.

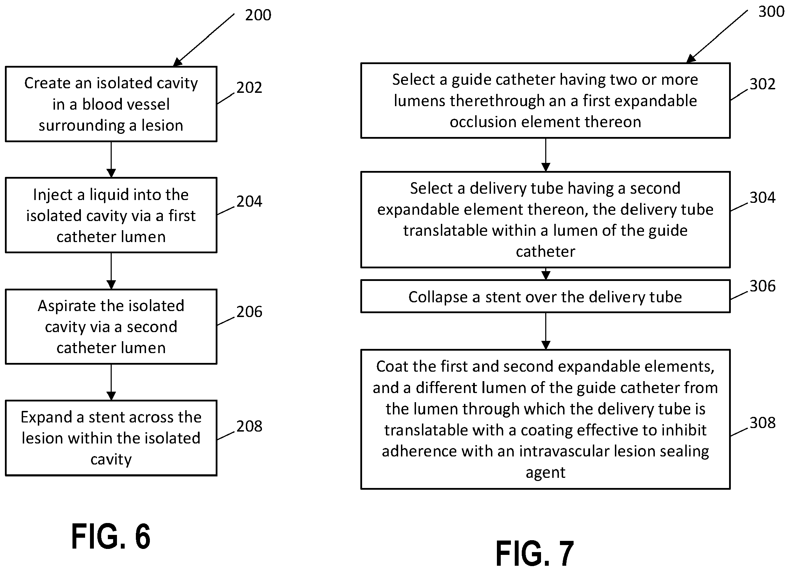

FIG. 6 is a flow diagram of a method 200 for treating an intravascular lesion. In step 202, an isolated cavity can be created in a blood vessel surrounding a lesion.

In step 204, a liquid can be injected into the isolated cavity via a first catheter lumen. The liquid can be an ICAD treatment agent designed to stabilize the lesion and/or form a seal against the lesion.

In step 206, the isolated cavity can be aspirated via a second catheter lumen. The second catheter lumen can be independent from the first catheter lumen.

In step 208, a stent can be expanded across the lesion within the isolated cavity.

FIG. 7 is a flow diagram of a method 300 for treating an intravascular lesion. In step 302 a guide catheter having two or more lumens therethrough and a first expandable occlusion element is selected. The guide catheter can be a balloon guide catheter as illustrated and described herein, a variation, thereof, or an alternative thereto as appreciated and understood by a person having ordinary skill in the art according to the teaching herein.

In step 304, a delivery tube translatable within a lumen of the guide catheter and having a second expandable element can be selected. The delivery tube can be a delivery tube 108a-c as illustrated and described herein, a variation, thereof, or an alternative thereto as appreciated and understood by a person having ordinary skill in the art according to the teaching herein.

In step 306, a stent can be collapsed over the delivery tube.

In step 308, the first and second expandable elements and a different lumen of the guide catheter than the lumen through which the delivery tube is translatable can be coated with a material effective to inhibit adherence of such components with an intravascular lesion sealing agent.

The descriptions contained herein are examples of embodiments of the invention and are not intended in any way to limit the scope of the invention. As described herein, the invention contemplates many variations and modifications of the intravascular treatment system, including alternative materials, alternative device structures, alternative treatment steps, etc. The scope of the invention is defined by the claims.

## Claims

1. An intravascular treatment system (100a) comprising:
a balloon guide catheter (102) comprising:
a distal end (124),
a proximal end,
a balloon (112) positioned approximate the distal end,
an inflation lumen (128) in communication with the balloon,
a drug delivery lumen (118), and
a device delivery lumen (116); and
a delivery tube (108a) sized to be positioned within the device delivery lumen of the balloon guide catheter, the delivery tube comprising an expandable occluding element (114a) thereon; and
wherein the system further comprises;
a stent (110a) surrounding the delivery tube (108a) and positioned in a proximal direction in relation to the expandable occluding element;
a sheath (106) surrounding the stent, wherein the sheath is positioned to inhibit the stent from expanding, and wherein the sheath is movable to allow the stent to expand, and wherein the sheath is positioned to inhibit the expandable occluding element from expanding, and wherein the sheath is movable to allow the expandable occluding element to expand.

2. The system of claim 1, wherein the stent (110a) is self-expandable.

3. The system of claim 2, wherein the expandable occluding element (114a) is self-expandable,

4. The system of claim 2, wherein the expandable occluding element (114a) is inflatable.

5. The system of claim 1, wherein the delivery tube (108a) further comprises an angioplasty balloon (126), and the stent surrounds the angioplasty balloon.

6. The system of claim 1, wherein the delivery tube (108a) and the balloon (112) on the balloon guide catheter (102) each respectively comprises a coating thereon effective to inhibit adherence with an intravascular lesion sealing agent.

7. The system of claim 1, wherein the drug delivery lumen (118) comprises a coating therein to inhibit adherence with an intravascular lesion sealing agent.

8. A method for constructing an intravascular treatment system (100a), the method comprising:
selecting a guide catheter (102) comprising a first expandable element (112) thereon, a first lumen (118), and a second lumen (116);
selecting a delivery tube (108a) sized to be positioned within the second lumen (116), the delivery tube comprising a second expandable occluding element (114a) thereon;
collapse a stent (110a) over the delivery tube;
positioning a sheath (106) over the stent to thereby inhibit expansion of the stent, wherein the sheath is movable to allow the stent to expand; and
positioning the sheath to inhibit the second expandable occluding element from expanding, wherein the sheath is movable to allow the second expandable occluding element to expand.

9. The method of claim 8, further comprising at least one of the following steps:
positioning an angioplasty balloon (126) under the stent

10. The method of claim 8 or claim 9, further comprising applying one or more coatings to the first expandable occluding element (112), the second expandable occluding element (114a), the stent (110a), and an interior of the first lumen (118), the one or more coatings effective to inhibit adherence with an intravascular lesion sealing agent.

## Patentansprüche

1. Intravaskuläres Behandlungssystem (100a) umfassend:
einen Ballonführungskatheter (102) umfassend:
ein distales Ende (124),
ein proximales Ende,
einen Ballon (H2), der nahe dem distalen Ende angeordnet ist,
ein Aufblählumen (128) in Kommunikation mit dem Ballon, ein Arzneimittelabgabelumen (118) und
ein Vorrichtungsabgabelumen (116); und
ein Abgaberohr (108a), bemessen innerhalb des Vorrichtungsabgabelumens des Ballonführungskatheters angeordnet zu werden, wobei das Abgaberohr ein expandierbares Absperrelement (114a) daran umfasst; und
wobei das System ferner umfasst:
einen Stent (110a), der das Abgaberohr (108a) umgibt und in einer proximalen Richtung bezogen auf das expandierbare Absperrelement angeordnet ist;
eine Hülle (106), die den Stent umgibt, wobei die Hülle angeordnet ist, um den Stent an Expandieren zu hindern, und wobei die Hülle beweglich ist, um dem Stent Expandieren zu ermöglichen, und wobei die Hülle angeordnet ist, um das expandierbare Absperrelement an Expandieren zu hindern, und wobei die Hülle beweglich ist, um dem expandierbaren Absperrelement Expandieren zu ermöglichen.

2. System gemäß Anspruch 1, wobei der Stent (110a) selbstexpandierbar ist.

3. System gemäß Anspruch 2, wobei das expandierbare Absperrelement (114a) selbstexpandierbar ist.

4. System gemäß Anspruch 2, wobei das expandierbare Absperrelement (114a) aufblähbar ist.

5. System gemäß Anspruch 1, wobei das Abgaberohr (108a) ferner einen Angioplastieballon (126) umfasst und der Stent den Angioplastieballon umgibt.

6. System gemäß Anspruch 1, wobei das Abgaberohr (108a) und der Ballon (112) an dem Ballonführungskatheter (102) jeweils eine Beschichtung darauf umfassen, die wirksam ist, Haftung mit einem intravaskuläre Läsionen abdichtenden Mittel zu hemmen.

7. System gemäß Anspruch 1, wobei das Arzneimittelabgabelumen (118) eine Beschichtung darin umfasst, um Haftung mit einem intravaskuläre Läsionen abdichtenden Mittel zu hemmen.

8. Verfahren zum Einrichten eines intravaskulären Behandlungssystems (100a), wobei das Verfahren umfasst:
Auswählen eines Führungskatheters (102) umfassend ein erstes expandierbares Element (112) daran, ein erstes Lumen (118) und ein zweites Lumen (116);
Auswählen eines Abgaberohrs (108a), bemessen zur Positionierung in dem zweiten Lumen (116), wobei das Abgaberohr ein zweites expandierbares Absperrelement (114a) daran umfasst;
Kollabieren des Stents (110a) über dem Abgaberohr;
Positionieren einer Hülle (106) über dem Stent, um dadurch Expansion des Stents zu verhindern, wobei die Hülle beweglich ist, um Expandieren des Stents zu ermöglichen; und
Positionieren der Hülle, um Expansion des zweiten expandierbaren Absperrelements zu verhindern, wobei die Hülle beweglich ist, um Expandieren des zweiten expandierbaren Absperrelements zu ermöglichen.

9. Verfahren gemäß Anspruch 8, ferner umfassend wenigstens einen der folgenden Schritte:
Positionieren eines Angioplastieballons (126) unter dem Stent.

10. Verfahren gemäß Anspruch 8 oder Anspruch 9, ferner umfassend Aufbringen einer oder mehrerer Beschichtungen auf das erste expandierbare Absperrelement (112), das zweite expandierbare Absperrelement (114a), den Stent (110a) und eine Innenseite des ersten Lumens (118), wobei die eine oder mehreren Beschichtungen wirksam sind, Haftung mit einem intravaskuläre Läsionen abdichtenden Mittel zu hemmen.

## Revendications

1. Système de traitement intravasculaire (100a) comprenant :
un cathéter de guidage à ballonnet (102) comprenant :
une extrémité distale (124),
une extrémité proximale,
un ballonnet (112) positionné à proximité de l'extrémité distale,
une lumière de gonflage (128) en communication avec le ballonnet,
une lumière d'administration de médicament (118), et
une lumière d'administration de dispositif (116) ; et
un tube d'administration (108a) dimensionné pour être positionné dans la lumière d'administration de dispositif du cathéter de guidage à ballonnet, le tube d'administration comprenant un élément d'occlusion extensible (114a) ; et
le système comprenant en outre :
une endoprothèse (110a) entourant le tube d'administration (108a) et positionnée dans une direction proximale par rapport à l'élément d'occlusion extensible ;
une gaine (106) entourant l'endoprothèse, la gaine étant positionnée pour empêcher l'endoprothèse de se dilater, et la gaine étant mobile pour permettre à l'endoprothèse de se dilater, et la gaine étant positionnée pour empêcher l'élément d'occlusion extensible de se dilater, et la gaine étant mobile pour permettre à l'élément d'occlusion extensible de se dilater.

2. Système selon la revendication 1, l'endoprothèse (110a) étant auto-extensible.

3. Système selon la revendication 2, l'élément d'occlusion extensible (114a) étant auto-extensible.

4. Système selon la revendication 2, l'élément d'occlusion extensible (114a) étant gonflable.

5. Système selon la revendication 1, le tube d'administration (108a) comprenant en outre un ballon d'angioplastie (126), et l'endoprothèse entourant le ballon d'angioplastie.

6. Système selon la revendication 1, le tube d'administration (108a) et le ballonnet (112) sur le cathéter de guidage à ballonnet (102) comprenant chacun respectivement un revêtement efficace pour inhiber l'adhérence avec un agent de scellement de lésion intravasculaire.

7. Système selon la revendication 1, la lumière d'administration de médicament (118) comprenant un revêtement permettant d'inhiber l'adhérence avec un agent de scellement des lésions intravasculaires.

8. Procédé de construction d'un système de traitement intravasculaire (100a), le procédé comprenant :
la sélection d'un cathéter de guidage (102) comprenant un premier élément extensible (112), une première lumière (118) et une seconde lumière (116) ;
la sélection d'un tube d'administration (108a) dimensionné pour être positionné dans la seconde lumière (116), le tube d'administration comprenant un second élément d'occlusion extensible (114a) ;
le repliement d'une endoprothèse (110a) sur le tube d'administration ;
le positionnement d'une gaine (106) sur l'endoprothèse afin d'empêcher la dilatation de l'endoprothèse, la gaine étant mobile pour permettre à l'endoprothèse de s'étendre ; et
le positionnement de la gaine pour empêcher le second élément d'occlusion extensible de se dilater, la gaine étant mobile pour permettre au second élément d'occlusion extensible de se dilater.

9. Procédé selon la revendication 8, comprenant en outre au moins l'une des étapes suivantes :
le positionnement d'un ballon d'angioplastie (126) sous l'endoprothèse.

10. Procédé selon la revendication 8 ou selon la revendication 9, comprenant en outre l'application d'un ou plusieurs revêtements sur le premier élément d'occlusion extensible (112), le deuxième élément d'occlusion extensible (114a), l'endoprothèse (110a), et un intérieur de la première lumière (118), le ou les revêtements étant efficaces pour inhiber l'adhérence avec un agent de scellement de lésion intravasculaire.
